(19) Europäisches Patentamt

European Patent Office

Office européen des brevets

(11) **EP 4 056 710 A1**

(12) **EUROPEAN PATENT APPLICATION**

(43) Date of publication:
**14.09.2022 Bulletin 2022/37**

(21) Application number: **21162266.7**

(22) Date of filing: **12.03.2021**

(51) International Patent Classification (IPC):
*C12Q 1/04* (2006.01)          *G01N 33/58* (2006.01)

(52) Cooperative Patent Classification (CPC):
**G01N 33/582; C12N 13/00; C12Q 1/04**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität für Bodenkultur Wien
1180 Wien (AT)**

(72) Inventors:
• **Zand, Elena
  1180 Wien (AT)**

• **Jäger, Henry
  1180 Wien (AT)**
• **Zunabovic-Pichler, Marija
  1180 Wien (AT)**
• **Schönher, Christoph
  1180 Wien (AT)**
• **Domig, Konrad
  1180 Wien (AT)**

(74) Representative: **Schwarz & Partner Patentanwälte
GmbH
Patentanwälte
Wipplingerstraße 30
1010 Wien (AT)**

(54) **METHOD FOR THE DETECTION OF PARTICLES**

(57)     The present invention relates to a method for determining the presence and/or amount of biotic and abiotic particles in a liquid sample by subjecting the sample to electroporation, wherein a fluorescent dye is added to the sample before or after electroporation and by detecting and/or quantifying the stained particles by flow cytometry.

EP 4 056 710 A1

**Description**

TECHNICAL FIELD

The present invention relates to the field of detecting particles in liquid or gaseous samples.

BACKGROUND ART

In order to monitor the hygienic status of air and/or water (i.e. wastewater, stagnant fluids) during the production of food, pharmaceuticals and biotechnological products or in medical facilities, a reliable method for determining and quantifying the presence of biotic particles such as bacteria, fungi and other airborne organisms is essential. The determination of total biotic particle count in samples is demanding, whereby the number of microorganisms is often underestimated. In addition, the differentiation between biotic and abiotic particles, especially for gaseous samples such as air, is important, since the presence of particles in general is not necessarily an indication whether a sample (e.g. air) is contaminated with biotic particles like pathogens.

Currently, cultivation-based analytical methods are used as a standard method to determine the presence of biotic particles within the food and biotechnology (bio)industry. However, these methods are limited to a few parameters and thus, the actual microbial dynamics are not completely covered. A major limitation includes the lack of cultivability and/or detection of microorganisms like bacteria within the viable but non-culturable (so called VBNC) state. Furthermore, these cultivation-based methods require a long incubation period of at least one to five days, which makes it impossible to react on time to avoid further contamination. At the same time, the complexity of bacterial colonies at the culture level is often not correctly assessed, as cultured microorganisms do not generally make up the largest proportion of microorganisms found within the food and bio industry.

In addition, cultivation-independent device-based technologies exist (e.g. molecular biological methods, biosensors or fluorescence-based methods) to quantify particles. However, such methods do not allow the differentiation between biotic (e.g. microbial) and abiotic particles and are therefore limited in their informative value with respect to the amount of microbial cells in a sample.

Known cultivation-independent fluorescence-based technologies to determine the presence of biotic particles in samples involve cell-based flow cytometry. This well-known technique can be used to determine, e.g., the overall microbiological status of liquid samples. Flow cytometry enables cultivation-independent quantification of microbial cells in a short time. Chen and Li (J. Environ. Monit. 2005, 7, 950-959) describe the application of flow cytometry combined with a fluorescent technique to determine and quantify the total concentration and viability of bioaerosols.

Currently, flow cytometric methods for research purposes are based on the use of toxic and/or mutagenic cell membrane permeable dyes to determine the total cell count (TCC) or intact cell count (ICC). For staining, the sample is typically incubated in the presence of the dye for a rather long period of time. After attachment of a DNA-intercalating dye to the nucleic acids of the cells in the incubation step, the dye unfolds a fluorescence signal, which is detected by various detectors.

However, for safety reasons, such toxic and mutagenic dyes are not suitable for applications within the food- and bioindustry. Therefore, a rapid, easy-to use and inparticular safe method is desired for the detection of particles in a sample. It is also desired to improve the sensitivity of current methods.

It is an object of the present invention to overcome the drawbacks of state of the art methods and to provide a rapid and sensitive easy-to-use method to determine the amount of biotic and abiotic particles in various samples, in particular originating from any food or biotechnological production facility.

SUMMARY OF THE INVENTION

The present invention provides a method for determining the presence and/or amount of biotic particles and abiotic particles in a liquid sample, said method comprising the steps of:

   a) subjecting a liquid sample to electroporation to obtain an electroporated liquid sample, wherein at least one fluorescent dye is added to the liquid sample before and/or after electroporation; and
   b) determining the presence and/or amount of biotic particles by detecting and/or quantifying particles labelled with said dye in the electroporated sample and determining the presence and/or amount of abiotic particles by detecting and/or quantifying particles not labelled with said dye in the electroporated sample,

wherein said particles are detected and/or quantified by flow cytometry.

The method of the present invention comprises a sample pre-treatment step in which biotic particles present in a sample are electroporated and stained with a dye (step a) followed by the detection of the stained and unstained particles by flow cytometry(step b).

It has surprisingly been found that electroporation is particularly useful for staining biotic particles present in a sample resulting in a simplified and fast method for determining biotic particles within a sample in a flow cytometric measurement. In particular, the electroporation and staining step according to the present invention provides a fast, non-destructive, selective sample pre-treatment at low operational costs and avoids inducing thermal effects in the sample.

Thus, the present invention discloses an optimization of the signal intensity in a flow cytometric analysis resulting from a sample pre-treatment step consisting of electroporation and staining with a fluorescent dye.

Electroporation before and/or after adding a fluorescent dye to the sample to stain biotic particles in a sample is particularly advantageous because it increases the permeability of the cell membranes for fluorescent dyes. This results in a surprisingly improved signal intensity and thus improved sensitivity when detecting stained cells (step b), e.g. when using flow cytometry as a detection method.

Using electroporation as a sample pre-treatment can significantly accelerate the time necessary for staining the particles and can at the same time increase the signal intensity of naturally permeable dyes in the detection step.

Additionally, the sample pre-treatment by electroporation surprisingly allows using a wide range of dyes for staining the particles. Therefore, both membrane-permeable dyes and non-permeable dyes are suitable for staining the particles.

Electroporation can be used for dyes which are not able to pass cell barriers without any additional pre-treatment step and thus, can be also used in the method of the present invention. This allows using dyes which are in principle not membrane permeable. With such dyes, the risk of undesired DNA intercalation can be avoided. Thereby, safety risks for the human operators of the analysis method can be significantly reduced by using such alternative dyes. Therefore, the present invention allows besides the use of state-of-the-art dyes the use of non-toxic fluorescent dyes for the staining (labelling) of biotic particles.

The inventive method is fast and accurate and allows to discriminate between biotic and abiotic particles and to determine the total amount of particles. Due to the fast staining and detection, the inventive method is particularly advantageous for the on-line monitoring of particles, e.g. contaminants.

In some embodiments, the liquid sample is obtained by contacting a gaseous fluid, such as air, with a liquid matrix. Thus, the present invention also allows analysing biotic and abiotic particles in gaseous samples. This is of particular advantage for applying the inventive method for hygiene monitoring or control in food- and biotechnological production processes.

In addition, the present invention relates to a method for discriminating between different types of biotic particles in a liquid sample comprising particles, said method comprising the steps of:

a) dividing the liquid sample into at least two subsamples;
b) subjecting each subsample of step a) to electroporation at a different electric field strength to obtain an electroporated liquid sample for each subsample, wherein at least one fluorescent dye is added to each subsample before or after electroporation;

c) detecting and/or quantifying biotic particles labelled with said dye in each electroporated subsample by flow cytometry; and
d) identifying in each subsample one or more types of biotic particles as the biotic particles labelled with said dye.

Therefore, the present invention allows to determine the types of biotic particles in a sample, e.g. to discriminate between yeast cells and bacterial cells.

BRIEF DESCRIPTION OF THE FIGURES

**Fig. 1** shows pulsed electric fields-assisted flow cytometry (PEF-assisted FCM) protocols for various dyes and staining methods.
**Fig. 2** shows plots of fluorescence intensity measured by flow cytometry for samples of *E. coli* cells stained with A: SYBR™ Green (SG1) and B: propidium iodide (PI).
**Fig. 3** shows plots of fluorescence intensity measured by flow cytometry for samples of *E. coli* cells stained with A: EvaGreen (eG) (left) and GelGreen (gG) (right) and B: GelRed (gR).

DESCRIPTION OF EMBODIMENTS

The term "biotic particles" relates to particles of biological origin, such as microorganisms including bacteria, fungal cells, yeast cells and microalgal cells. In contrast, "abiotic particles" are particles which are not of biological origin, such particles comprising organic, e.g. chemical contaminants, or inorganic molecules, e.g. dust particles.

According to a preferred embodiment of the present invention, said biotic particles comprise one or more of the group consisting of bacterial cells, bacterial spores, fungal cells, preferably yeast cells or mold, fungal spores and microalgae. The present invention is particularly useful to detect and quantify bacteria, fungi, yeast and microalgae, which are common contaminants in food and biotechnological production.

The term "electroporation" as used herein refers to the use of pulsed electric fields (PEF) to induce microscopic pores, in the cell membranes. In principle, reversible (temporary pore formation) or irreversible electroporation (permanent pore formation, which is associated with cell death) can occur. The stability, number and size of the pores created depends on the applied electric field strength level as well as on the amplitude and duration of the pulse(s). Most important process parameters comprise the specific energy input, the electric field strength level, pulse shape, - width and -repetition rate as well as the treatment temperature and the electric field exposure time. To obtain pores, a certain threshold limit has to be surpassed, which varies between the different cell types.

With the present invention, loading of cells with molecules/permeable fluorescent dyes can be fastened up and as well as the staining intensity can be increased. Additionally, this invention allows molecules which are not normally membrane permeable to pass from the extracellular environment into the cells. In addition, the entering of membrane permeable molecules into the cell is accelerated and improved with the inventive method.

A fluorescent dye according to the present invention can enter the cell or attach to the cell membrane. Herein, this process is referred to as "labelling" or "staining".

According to a preferred embodiment of the present invention, said electroporation is performed at an electric field strength of between 0.5 and 50 kV/cm, preferably between 8 and 50 kV/cm, more preferably between 8 and 30 kV/cm. If the electric field strength is within this range, optimal electroporation and quantitative staining of the biotic particles with the fluorescent dye can be achieved.

The term "fluorescent dye" or "fluorescent stain" is to be understood as an organic substance which exhibits fluorescence, i.e. which absorbs light at a first wavelength and emits light at a second wavelength that is longer than the first wavelength.

According to the present invention, the fluorescent dye is any fluorescent dye suitable for labelling biotic particles. The dye may be a membrane-permeable or non-permeable dye. Preferably, the fluorescent dye is a non-toxic dye. As used herein, the term "non-toxic" or "non-hazardous" refers to a dye, which is non-(cyto)toxic and/or non-mutagenic in the applied concentration. Preferably, no special disposal and no special safety equipment for handling the dye in the laboratory is required. According to the present invention, the non-toxic dye is not cell membrane permeable. Thus, a "non-toxic" dye according to the present invention is not membrane-permeable (i.e. non-permeable), not (cyto)toxic and/or not mutagenic.

The term "membrane permeable" refers to the ability of the dye to permeate a cell membrane and/or cell wall and to internalize into cells.

According to one embodiment, the non-toxic fluorescent dye is a DNA-binding fluorescence dye. In a preferred embodiment, the non-toxic DNA-binding fluorescent dye is selected from the group consisting of Eva-Green™, Gelgreen™, Gelred™, POPO-1™, CellTox Green™, DRAQ7™ and RedDot2™.

Gelgreen™ consists of two acrine orange subunits (IUPAC name: 10,10'-(6,22-dioxo-11,14,17-trioxa-7,21-di-azaheptacosane-1,27-diyl)bis (3,6-bis (dimethylamino)acr idin-10-ium) iodide)). Gelred™ consists of two ethidium subunits (IUPAC name: 5,5'-(6,22-dioxo-11,14,17-trioxa-7,21-di-azaheptacosane-1,27-diyl)bis(3,8-diamino-6-phenylphenan-thridin-5-ium) iodide)).

In another preferred embodiment the non-toxic DNA-binding fluorescent dye is a cyanine dye or derivative thereof. The term "cyanine dye" as used herein refers to tetramethylindo(di)-carbocyanines. Preferably, the cyanine dye is selected from the group consisting of SYTOX Green™, SYTOX Blue™, SYTOX Orange™, YOYO-1™, YOPRO-1™ and BOBO-3™.

YOYO-1™ is a monomethine cyanine dye (IUPAC name: {1,1'-(4,4,8,8-tetramethyl-4,8-diazaundecamethylene)bis[4-[(3-methylbenzo-1,3-oxazol-2-yl)methylidene]-1,4-dihydro-quinolinium] tetraiodide}). YOPRO-1™ is a carbocyanine dye and BOBO-3™ is a dimeric cyanine stain.

The present invention is not limited to these exemplary lists of dyes.

According to the present invention, the fluorescent dye may be added to the sample prior to electroporation or after electroporation. Alternatively, the fluorescent dye may be added both prior and after the electroporation. In one embodiment, the fluorescent dye is added after electroporation, so that the dye can enter the electroporated cells. This may be particularly useful in continuous processes, where the sample is directly subjected to flow cytometry after the electroporation and staining step. Thereby, a biotic particle labelled with the fluorescent dye is obtained. The fluorescence of the biotic particle labelled with said dye is then used for determining the presence and/or amount of biotic particles.

According to the present invention, the biotic particles are detected and or quantified by flow cytometry.

The term "flow cytometry" as used herein refers to a technique for counting and detecting particles, based on scattered light and fluorescence signals. Briefly, a beam of light (usually laser light) is directed onto a hydrodynamically focused stream of fluid and scattered light as well as fluorescence of the particles are induced at the interrogation point. The forward (FSC) and side scatter (SSC) give information about the particles size and granularity, making it useful to analyse the morphology of i.e. microbial cells. After excitation of particles labelled with a fluorescent dye, or rather a fluorochrome, a fluorescent signal appears and thereby particles can be detected and quantified. Some particles also show natural fluorescence.

Using flow cytometry for the detection is particularly advantageous since it allows a fast and accurate detection of particles, which is superior over other methods for the detection of fluorescence, such as fluorescence microscopy.

According to a preferred embodiment, the inventive method is performed continuously. This allows an online monitoring of samples comprising particles, for example for real-time analysis of potential contaminants in food- and/or biotechnological production processes. Thus, hygiene monitoring and control can be accelerated and simplified. According to the present invention, continuous flow cytometric sample anaysis is possible due to the combined electroporation and staining step, which is significantly faster than staining by incubation (i.e. without electroporation), as commonly performed in the state of the art.

The total amount of particles in the sample can be determined by using scattered light detection. From the total amount of particles and the amount of biotic particles labelled with the dye, the amount of abiotic particles can be easily determined as the difference between the total amount of the particles and the biotic particles labelled with said dye. Additionally, the dye according to the present invention shows high specificity towards nucleic acids, and thus, interfering signals/background signals obtained from unspecific binding to other particles/abiotic particles are rare.

In the present invention, the amount of biotic particles is determined as the total cell count of biotic particles. The term "total cell count" refers to the sum of viable and non-viable cells, i.e. to the sum of the biotic particles whose cell membrane is still intact after electroporation and of biotic particles, whose cell membrane is injured/damaged due to electroporation or has already been damaged previous to electroporation.

According to a preferred embodiment, the liquid sample is filtrated prior to step a) of the inventive method. The sample is preferably filtered to remove very large particles that might interfere with flow cytometry. For example, a filter with a mesh size ranging from 10 to 100 $\mu$m, preferably from 20 to 80 $\mu$m, more preferably from 25 to 50 $\mu$m, may be used. In a particular preferred embodiment of the present invention the filter is a membrane filter comprising or consisting of a material which does not bind biotic cells like polyvinylidene fluoride, polyesters etc. One or more filters with a mesh size within the above ranges may be used, for example a coarse filter and a fine filter.

According to a specific embodiment of the present invention, the liquid sample is obtained by contacting gaseous fluid comprising particles with a liquid matrix. Thus, the present invention also allows analysing biotic and abiotic particles in gaseous fluids such as air. This is particularly advantageous for hygiene monitoring or control in food- and biotechnological production processes. In this embodiment, the gas (such as air) is collected and introduced into a liquid matrix to obtain the liquid sample according to the present invention, which is subjected to electroporation, staining and analysis.

The gaseous fluid may be collected using a cyclone or an impinger. For example, a cyclone air sampler as described by E. Carvalho et al. (Aerobiologia 2008, 24, 191-201) can be used. Gases may also be sampled using an impinger as for example described by F. Masotti et al. (Trends in Food Science & Technology 2019, 90, 147-156).

According to a preferred embodiment, the liquid sample comprises or consists of a buffer solution, preferably an aqueous buffer solution. A buffer solution helps to stabilize the pH value of the sample and to stain the particles.

For the buffer solution, a phosphate-buffered saline solution may be used, for example a buffer comprising sodium phosphate monobasic monohydrate and sodium phosphate dibasic heptahydrate and water, preferably deionized or distilled water; or sodium chloride and anhydrous dibasic sodium phosphate water, preferably deionized or distilled water. For example, the buffer solution may have the following composition: NaCl (8 g/L); KCl (0.2 g/L), $Na_2HPO_4$ (1.44 g/L), $KH_2PO_4$ (0.24 g/L).

Optionally, the buffer solution may comprise further components. According to one embodiment of the present invention, the buffer solution further comprises one or more of a chelator such as ethylenediamine tetraacetic acid, a preservative such as $NaN_3$, fetal bovine serum, a surfactant and/or an antifoaming agent.

Ethylenediaminetetraacetic acid (EDTA) acts as chelator and can lower cation-dependent cell to cell adhesion, which is useful for accurate analysis by flow cytometry. A preservative such as $NaN_3$ is useful to stabilize the solution. Fetal bovine serum (BSE) can be used for minimizing non-specific binding of the dye.

For sampling and staining of biotic particles from gaseous fluids, such as air samples, a surfactant can be added to lower the surface tension between i.e. gas and liquid. Examples for such surfactants comprise Tween® solutions, like Tween-20®, Tween-80®. Moreover, an antifoaming agent may be added to avoid foaming, such as an aqueoussilicone solution, line Ex-cell® Antifoam, Antifoam Y-30®.

For the electroporation step, the electrical conductivity of the liquid sample is important. The electrical conductivity is determined mainly by the buffer solution. According to a preferred embodiment of the present invention, the electrical conductivity of the buffer solution is between 0.1 and 20 mS/cm, preferably between 1 and 20 mS/cm, even more preferably between 3 and 15 mS/cm, even more preferably between 3 and 10 mS/cm.

In particular, staining and flow cytometric analysis is preferable with an electrical conductivity between 1 to 20 mS/cm, to avoid cell damage, e.g. due to osmotic pressure.

For example, a phosphate-buffered saline solution with an electrical conductivity of 16 mS/cm may be used.

Additionally, also the pH value of the liquid sample is important. According to a preferred embodiment, the pH value of the liquid sample is between 5 and 9, preferably between 6 and 8, more preferably around 7. A pH of the sample in this range, preferably a pH of around 7, allows an optimal analysis of the particles labelled with the fluorescent dye.

Another aspect of the present invention relates to a method for discriminating between different types of biotic particles in a liquid sample comprising particles, said method comprising the steps of:

a) dividing the liquid sample into at least two subsamples;

b) subjecting each subsample of step a) to electroporation at a different electric field strength to obtain an electroporated liquid sample for each subsample, wherein at least one fluorescent dye is added to each subsample before or after electroporation;

c) detecting and/or quantifying biotic particles labelled with said dye in each electroporated subsample by flow cytometry; and

d) identifying in each subsample one or more types of biotic particles as the biotic particles labelled with said dye.

A "subsample" as used herein is to be understood as a part of the liquid sample. The liquid sample may for example be divided into at least two, three, four, five, six, seven, eight, nine or ten subsamples. For each subsample, the electroporation (step a) and staining step (step b) as well as the detection step (step c) is performed separately and independently from the other subsamples.

Depending on the type of biotic particles, the pores in the cell membranes are formed at different electric field strengths. This can be used to distinguish between different types of biotic particles, since only particles undergoing electroporation above a critical/specific threshold level are labelled with the fluorescent dye. Accordingly, in each subsample one or more types of biotic particles as the biotic particles labelled with said dye can be identified.

The following approximate electric field strength ranges are given for the electroporation of biotic particles according to the present invention:

- Yeasts: 4-15 kV/cm
- Gram-negative bacteria: 5-30 kV/cm (preferable range around 20-25 kV/cm)
- Gram-positive bacteria: 11-40 kV/cm (irreversible electroporation starting at around 30 kV/cm)
- Microalgae: 10-50 kV/cm (irreversible electroporation starting at around 20-25 kV/cm)

It is noted that these absolute values are approximated values, which may vary slightly, depending on the individual species and environment. The skilled person is aware of suitable electroporation conditions depending on the type of microorganisms, as for example listed in the Handbook of Electroporation (edited by D. Miklavcic, Springer International Publishing, 2017).

Thus, yeast cells are electroporated at lower electric field strengths than gram-negative bacteria, which are in turn electroporated at lower electric field strengths than gram-positive bacteria, microalgae and/or molds.

Thus, according to one embodiment of the present invention, yeast cells are detected and/or quantified if said electroporation is performed at an electric field strength between 4-15 kV/cm.

According to another embodiment, gram-negative bacterial cells are detected and/or quantified if said electroporation is performed at an electric field strength between 5 and 30 kV/cm.

According to another embodiment, gram-positive bacterial cells are detected and/or quantified if said electroporation is performed at an electric field strength between 11 and 40 kV/cm.

According to another embodiment, microalgae are detected and/or quantified if said electroporation is performed at an electric field strength between 10 and 50 kV/cm.

According to another embodiment, molds and/or mold spores can be detected and/or quantified if said electroporation is performed at an electric field strength between 10 and 50 kV/cm.

It is expected that at electric field strengths below 5 kV/cm, gram-negative bacteria, gram-positive bacteria, microalgae and molds are not electroporated and below 11 kV/cm, gram-positive bacteria are not electroporated. Below 10 kV/cm, gram-positive bacteria, microalgae and molds are not electectroporated.

Therefore, performing the electroporation at an electric field strength of around 4 kV/cm would selectively electroporate yeast cells. Performing the electroporation below 10 kV/cm would electroporate yeast cells and a majority of gram-negative bacteria.

Thereby, different types of biotic particles in a sample can be distinguished. Thus, the present invention is for example advantageous to determine whether a contamination in a sample from food- or biotechnological production stems from yeast, bacteria or molds and microalgae.

DETAILED DESCRIPTION OF THE FIGURES

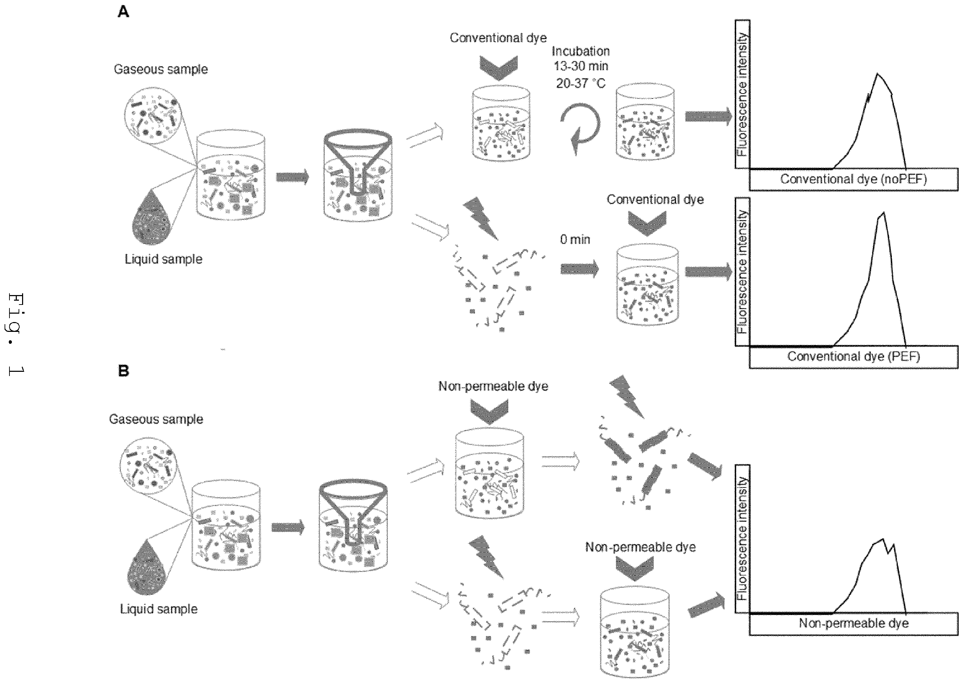

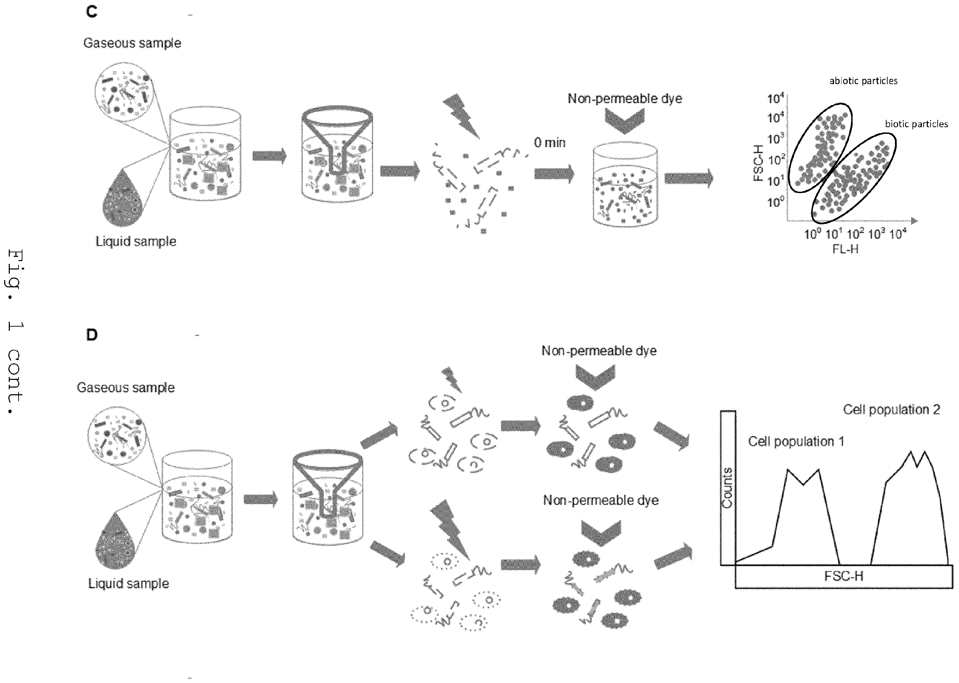

**Fig. 1** shows pulsed electric fields-assisted flow cytometry (PEF-assisted FCM) protocols for A: the acceleration of the method and increase in mean fluorescence intensity (MFI) of conventional fluorescent dyes, B: the use of alternative (non-permeable, non-toxic) fluorescent dyes, C: the differentiation of biotic particles, preferably microbial cells, to abiotic particles, and D: the differentiation among different microbial populations, i.e., among gram-negative bacteria and yeasts. In protocol B, staining prior to PEF-treatment and staining after PEF-treatment is shown, as both methods can be used. For A, B and D, only staining after PEF-treatment is depicted, but also staining prior to PEF-treatment is possible. Abbreviations: FSC-H (Forward Scatter), FL-H (fluorescence spectra).

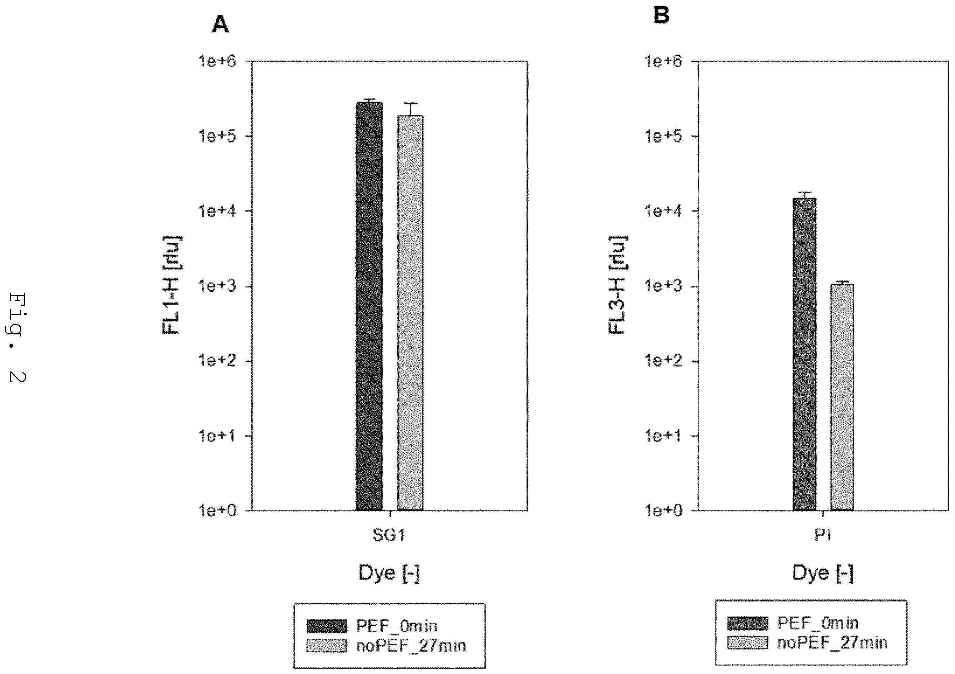

**Fig. 2** shows plots of fluorescence intensity (green fluorescence: FL1-H; red fluorescence: FL3-H) measured by flow cytometry for samples of *E. coli* cells pre-treated by PEF ("PEF_0min) and not pre-treated ("noPEF_27min"). Staining was performed with A: SYBR™ Green (SG1) and B: propidium iodide (PI). Abbreviations: rlu (relative light units).

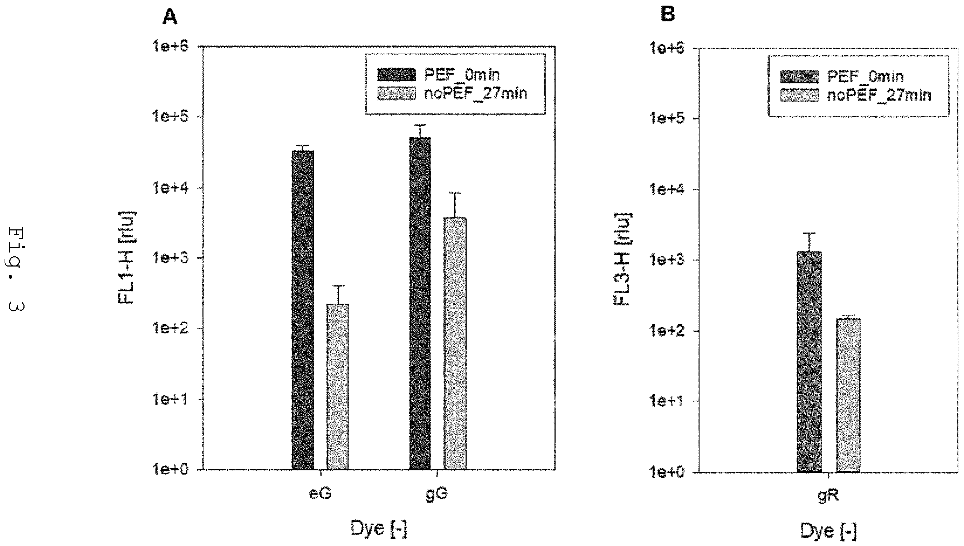

**Fig. 3** shows plots of fluorescence intensity measured by flow cytometry for samples of *E. coli* cells pre-treated by PEF ("PEF_0min) and not pre-treated ("noPEF_27min") . Staining was performed with alternative (i.e. non-permeable, non-toxic) dyes A: EvaGreen (eG) (left) and GelGreen (gG) (right), which show fluorescence in the green spectrum (FL1-H) and B: GelRed (gR), which shows fluorescence in the red spectrum (FL3-H). Abbreviations: rlu (relative light units).

EXAMPLES

**Example 1:**

Microbial growth conditions

Escherichia coli ATCC 9637 was used as a model microorganism. For initial cultivation from cryo cultures (-80 °C), a loopful of the stock culture was streaked out on tryptic soy agar plates (TSA; VWR International bvba, Belgium), supplemented with 0.6 % (w/v) yeast extract (YE; Carl Roth GmbH, Germany) and further incubated overnight at 37 °C. A single *E. coli* colony was taken from the TSAYE plates and inoculated in TSBYE (VWR) at 37 °C (aerobically). The culture was then standardized to an optical density at 600 nm ($OD_{600}$) of ~0.1 and incubated for 3.5 h at 37 °C to reach the early stationary growth phase, as previously determined by $OD_{600}$ measurements. As buffer solution, in $dH_2O$ diluted PBS (PBS Roti® fair 7.4; Carl Roth GmbH, Germany) with an electrical conductivity of 3.0 mS/cm (pH 7.30) was used. The cells were harvested and washed in this PBS solution by centrifugation at 3200 x g for 15 min at ambient temperature. The supernatant was discarded, and the *E. coli* suspension was resuspended 1:15 in the respective buffer solution to obtain a final cell concentration of $N_0 \sim 10^7$ colony forming units (CFU) per ml, which was further used for the electroporation trials.

Pulsed electric fields (PEF) treatment setup and treatment parameters

A 6 kW pulse modulator (ScandiNova Systems AB, Sweden), was used for PEF experiments. The modulator allows the delivery of mono or bipolar square wave pulses and can be operated with voltages up to 25 kV, currents up to 200 A, frequencies up to 1000 Hz, and pulses from 1-5 μs. Voltage and current measurement, monitoring of pulse shapes and temperature measurements (including inlet and outlet temperature) were conducted as described by Schrottrof et al. (Journal of Food Engineering 2019, 243, 142-152).

A continuous co-linear treatment chamber, consisting of a single central cylindrical stainless steel high-voltage electrode as well as two cylindrical Teflon® insulators surrounded by two ground electrodes, was used for experimental trials. Dimensions of the treatment chamber are described in previous work (Reineke et al., Frontiers in Microbiology 2015, 6, 400). A laminar mass flow of 83.3 ml/min ($Re_{max}$ of 442.1) as well as a mean residence time in the electric field zone of 71.8 ms was obtained by using a screw pump (MDC 006-12, Seepex GmbH, Germany) . A $C_{chamber}$ factor of 1.6/cm was used to calculate the electric field strength (E) (Eq. 1), as defined by Jaeger et al. (Innovative Food Science & Emerging Technologies 2009, 10(4), 470-480):

$$E = C_{chamber} \quad U \quad [V] \qquad \text{Eq. 1}$$

The specific energy input ($W_{spec}$) was calculated based on the energy dissipated per pulse ($W_{pulse}$), the pulse repetition rate (f) as well as the mass flow rate ($\dot{m}$) (Eq. 2), whereof $W_{pulse}$ is calculated including the pulse width ($\tau$), voltage (U) and current (I) (Eq. 3):

$$W_{spec} = \frac{W_{pulse} \, f}{\dot{m}} \, [\frac{J}{kg}] \qquad \text{Eq. 2}$$

$$W_{pulse} = \quad U \, I \, \tau \, [\frac{J}{kg}] \qquad \text{Eq. 3}$$

PEF pre-treatment of liquid sample

For PEF pre-treatment a maximum electric field strength of 20 kV/ was used. 20 kV/cm is above the critical electric field strength ($E_{crit}$) for *E. coli,* thus resulting in membrane permeabilization and irreversible electroporation of *E. coli* cells at neutral pH. Before PEF pre-treatment, the system was rinsed with a buffer solution showing the same electrical conductivity as the liquid sample, and parameters were set to allow the targeted $W_{spec}$ level. The buffer solution was replaced with the *E. coli* suspension as soon as stable conditions were reached. Collection of the sample was carried out after 5 min, based on a residence time distribution profile. The untreated reference was collected afterward when the generator was switched off, at the outlet of the PEF equipment. Once the samples were collected, the reference and PEF-treated samples were kept on ice until further flow cytometric analysis.

Staining procedure and particle detection with flow cytometry

Samples were treated by electroporation prior to flow cytometry anaylsis ("inventive method"). For control purposes, staining was performed without electroporation pre-treatment by incubating the cells with a fluorescent dye for at least 13 minutes ("conventional method"). Prior to staining and flow cytometry analysis, PEF-treated samples (according to the invention) and reference samples (i.e. samples not treated by PEF, according to the conventional method) were diluted to obtain a final concentration ~$10^5$ cells/mL. Depeding on the PEF setup, staining can be performed prior to or after PEF treatment. When using the continuous PEF setup (as described above), staining after PEF-treatment was performed. PEF-treated and reference samples were kept on ice after PEF treatment until staining and flow cytometry analysis, in order to prevent cell membrane repair.

The *E. coli* sample and respective fluorescent dye were pipetted into a well of a 96-well microtiter plate to obtain a final volume of 150 μl. For comparison of conventional (i.e. hazardous and membrane permeable dyes) and alternative dyes (i.e. non-toxic, non-membrane permeable dyes), the optimal concentrations according to the manufacturers were used, as defined hereafter. SYBR® Green 1 (SG1; cell membrane permeable; Life Technologies, United States) was used as a respective "conventional" permeable and hazardous (referring to toxicity and mutagenicity) dye in the green fluorescence spectrum. Propidium iodide (PI; cell membrane impermeant; Thermo Fisher Scientific, United States) was used as a respective "conventional" non-permeable hazardous dye in the red fluorescence spectrum. Gel-Green® (gG; Biotium, Inc., United States) and Eva-Green® (Biotium) were used as alternative (i.e. non-permeable non-toxic) dyes in the green fluorescence spectrum, while GelRed® (Biotium) was considered as an example of an alternative dye in the red fluorescence spectrum.

For staining with SG1, gG, and gR, a final concentration of 1 x in 10 mM TRIS buffer (pH 8.1) was added to previously diluted samples. For eG a final concentration of 0.5 x in 10 mM TRIS buffer was added to the sample. For PI and cFDA, a final concentration of 6 μM or 10 μM in 10 mM TRIS buffer was added, respectively. For additional experiments, the final concentration of the alternative fluorescence dyes was optimized with the aim to allow a similar fluorescence intensity of alternative (gG, eG, gR) to conventional dyes (SG1, PI) in the respective fluorescence spectrum (green or red).

PEF-treated and untreated reference samples were stained with conventional and alternative dyes as described above and either measured immediately after staining (0 min of incubation time, PEF-treated samples) or incubated for 27 min (non PEF-treated samples) and analyzed. A simplified protocol for this method is shown in Fig. 1 A.

For optimal comparability of the conventional flow cytometry method (27 min incubation time without prior PEF-treatment) and the PEF-assisted flow cytometry method (0 min incubation time and prior PEF-treatment of bacterial cells), staining protocols were conducted at the same temperature (22.3 ± 0.2 °C). To allow the detection of both, bacterial cells with intact cell membranes and damaged cell membranes, by using one single alternative dye (see above), either staining prior to PEF-treatment or directly after PEF-treatment can be performed to load microbial cells with the dye. A simplified protocol for this method is shown in Fig. 1 B.

For flow cytometry analysis, a BD Accuri C6 flow cytometer (BD Life Sciences, USA), equipped with a 50 mW laser emitting at a wavelength of 488 nm was used. An injection volume of 25 μl as well as a flow rate of 0.07 ml/min was set. Two fluorescence signals (FL1, green fluorescence; FL-3, red fluorescence) and two light scattering signals (FSC-H, forward scatter; SSC-H, side scatter) were collected. A threshold of 8000 was applied for FSC-H. To avoid cross-contamination, a washing step with Milli-Q® water (100 μl injection volume) was conducted after each sample. FCM data were analyzed using the R statistical environment (version 3.6.1) and the flowCore package (version 1.52.1). The signal of the bacteria was electronically gated from background noise and fluorescence intensity in the red and green spectrum were used to compare PEF-treated (also referred to PEF-assisted) to reference (also referred to conventional) flow cytometry based detection method. Analyses were performed as biological and technical triplicates.

Results

Fig. 2 and Fig. 3 show an increase in fluorescence intensity for samples which were treated by PEF before the flow cytometry analysis compared to samples not treated by PEF. Among well known (i.e. conventional) dyes for flow cytometric measurements (SYBR™ Green (SG1) and propidium iodide (PI), Fig. 2), PEF pre-treatment allows to accelerate *E. coli* detection and increase green and red mean fluorescence intensity (FL1-H; FL3-H). The effect is in particular very well visible for PI, which is a membrane impermant dye.

Fig. 3 demonstrates the positive effect of the PEF-pre-treatment on the signal intensity for the alternative dyes EvaGreen (eG), GelGreen (gG) and GelRed (gR).

**Example 2: Differentiation of abiotic and biotic particles**

To differentiate between biotic and abiotic particles, air comprising particles was collected at 300 L/min for 10 minutes within a collection fluid (Coriolis® Collection liquid®; Bertin Technologies GmbH, France) by using the Coriolis® μ Air Sampler (Bertin Technologies GmbH). The remaining collection fluid after sampling was pipetted into sterile tubes and filtered with a 30 μm pre-separation filter (Miltenyi Biotec B.V. & Co. KG, Germany) to remove large particles that could disturb FCM analysis. The sample was then centrifuged at 3200 x g for 15 min at ambient temperature. The supernatant was discarded, and the pellet, together with a defined *E. coli* suspension ($10^5$ cells/ml) was resuspended in the respective buffer solution, which was further used for the electroporation trials.

The PEF pre-treatment was performed as described in above. Staining was performed with an alternative dye as described above, and immediately analyzed by flow cytometry, using the set-up described in above.

The FSC-H was used to detect abiotic and non-fluorescent particles. The subpopulation of *E. coli* cells was distinguished from other microorganisms within the air sample, by their difference in fluorescence signal and intensity. A simplified protocol for this method is graphically displayed in Fig. 1 C.

## Example 3: Differentiation of microbial subpopulations

The proof-of-concept for the differentiation of microbial subpopulations was performed as follows. *E. coli* subpopulation was grown as described above. Additionally, *Saccharomyces cerevisiae* ATCC 60236 cultivation and overnight culture was performed, as described by Schottroff et al., Innovative Food Science & Emerging Technologies, 2020, 63, 102372. Each cell suspension was adjusted to a cell concentration of ~$10^5$ cells/ml, based on OD measurement. Each suspension was subsequently centrifuged at 3200 x g for 15 min at ambient temperature, supernatants were removed and pellets were resuspended in the respective buffer media. In the following step, the two cell suspensions were combined and vortexed, to obtain a mixed culture of *E. coli* and *S. cerevisiae* cells with equal cell concentrations. The mixed culture was then divided into two equal subsamples, which were used for further PEF-treatment.

One of those subsamples was PEF treated as described above, with the PEF set up described above. The other subsample was PEF-treated at ≤ 12 kV/cm, preferably at 5 - 12 kV/cm, instead of 20 kV/cm. The remaining process parameters remained unmodified. Staining was performed with an alternative dye for each subsample, as described above, and then both subsamples were immediately analyzed by flow cytometry, using the FCM set-up described above. Within the subsample treated at ≤ 12 kV/cm, the *S. cerevisiae* cells showed a distinct higher fluorescence intensity compared to *E. coli* cells, while in the subsample treated at 20 kV/cm, both, *E. coli* and *S. cerevisiae,* showed a fluorescence signal. Based on these two subsamples, *S. cerevisiae* can be easily gated from the remaining *E. coli* cells. A simplified protocol of this method is illustrated in Fig. 1 D.

## Claims

1. A method for determining the presence and/or amount of biotic and abiotic particles in a liquid sample, said method comprising the steps of:

   a) subjecting a liquid sample to electroporation to obtain an electroporated liquid sample, wherein at least one fluorescent dye is added to the liquid sample before and/or after electroporation; and
   b) determining the presence and/or amount of biotic particles by detecting and/or quantifying particles labelled with said dye in the electroporated sample and determining the presence and/or amount of abiotic particles by detecting and/or quantifying particles not labelled with said dye in the electroporated sample,

   wherein said particles are detected and/or quantified by flow cytometry.

2. A method for discriminating between different types of biotic particles in a liquid sample, said method comprising the steps of:

   a) dividing the liquid sample into at least two subsamples;
   b) subjecting each subsample of step a) to electroporation at a different electric field strength to obtain an electroporated liquid sample for each subsample, wherein at least one fluorescent dye is added to each subsample before or after electroporation;
   c) detecting and/or quantifying biotic particles labelled with said dye in each electroporated subsample by flow cytometry; and
   d) identifying in each subsample one or more types of biotic particles as the biotic particles labelled with said dye.

3. The method of claim 1 or 2, wherein said liquid sample is obtained by contacting a gaseous fluid comprising particles with a liquid matrix, wherein said gaseous fluid is preferably air.

4. The method of any one of claims 1 to 3, wherein said fluorescent dye is a non-toxic fluorescent dye.

5. The method of any one of claims 1 to 4, wherein said liquid sample is filtrated prior to step a).

6. The method of any one of claims 1 to 5, wherein said biotic particles comprise one or more particles selected from the group consisting of bacterial cells, bacterial spores, fungal cells, preferably yeast cells or molds, fungal spores and microalgae.

7. The method of any one of claims 1 to 6, wherein said fluorescent dye is selected from the group consisting of EvaGreen™, Gelgreen™, Gelred™, POPO-1™, CellTox Green™, DRAQ7™ and RedDot2™, SYTOX Green™, SYTOX Blue™, SYTOX Or-ange™, YOYO-1™, YOPRO-1™ and BOBO-3™.

8. The method of any one of claims 3 to 7, wherein said gaseous fluid is collected using a cyclone or an impinger.

9. The method of any one of claims 1 to 8, wherein said liquid sample and/or the liquid matrix comprises or consists of a buffer solution.

10. The method of claim 9, wherein said buffer solution is a phosphate-buffered saline solution, wherein said buffer solution preferably further comprises one or more of a chelator such as ethylenediamine tetraacetic acid, a preservative such as $NaN_3$, fetal bovine serum, a surfactant and/or an antifoaming agent.

11. The method of any one of claims 1 to 10, wherein the electrical conductivity of the liquid sample is between 0.1 to 20 mS/cm, preferably between 1 to 15 mS/cm.

12. The method of any one of claims 1 to 11, wherein the pH value of the liquid sample is between 5 and 9, preferably between 6 and 8, more preferably around 7.

13. The method of any one of claims 1 to 12, wherein the electroporation in step a) is performed at an electric field strength between 0.5 and 50 kV/cm, preferably between 12 and 50 kV/cm, more preferably between 8 and 30 kV/cm.

14. The method of any one of claims 1 to 13, wherein step a) and step b) and liquid sample taking are performed continuously.

15. The method of any one of claims 1 to 14, wherein yeast cells are detected and/or quantified if said electroporation is performed at an electric field strength between 4-15 kV/cm and/or wherein gram negative bacterial cells are detected and/or quantified if said electroporation is performed at an electric field strength between 5 and 30 kV/cm, and/or wherein gram positive bacterial cells are detected and/or quantified if said electroporation is performed at an electric field strength between 11 and 40 kV/cm and/or wherein microalgae are detected and/or quantified if said electroporation is performed at an electric field strength between 10 and 50 kV/cm.

Fig. 1

Fig. 1 cont.

Fig. 2

Fig. 3

Europäisches
Patentamt

European
Patent Office

Office européen
des brevets

# EUROPEAN SEARCH REPORT

Application Number

EP 21 16 2266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | CHEN PEI-SHIH ET AL: "Bioaerosol characterization by flow cytometry with fluorochrome", JOURNAL OF ENVIRONMENTAL MONITORING, vol. 7, no. 10, 1 January 2005 (2005-01-01), page 950, XP055836866, ISSN: 1464-0325, DOI: 10.1039/b505224f | 1,3-15 | INV. C12Q1/04 G01N33/58 |
| A | * abstract * * page 950, column 1, paragraph 3 - column 2, paragraph 2 * * page 951, column 2, paragraph 3 - page 952, column 1, paragraph 1 * * page 954, column 1, paragraph 3 - column 2, paragraph 1 * * page 957, column 1, paragraph 2 - page 958, column 2, paragraph 3 * ----- | 2 | |
| Y | US 2006/172315 A1 (ANDERSON AMY L [US] ET AL) 3 August 2006 (2006-08-03) | 1,3-15 | |
| A | * paragraphs [0005], [0008], [0014], [0016], [0017], [0078] - [0080]; claim 24 * ----- | 2 | TECHNICAL FIELDS SEARCHED (IPC) C12Q G01N |
| Y | US 2014/127717 A1 (DIWU ZHENJUN [US] ET AL) 8 May 2014 (2014-05-08) * paragraphs [0386], [0389], [0390] * ----- | 1,3-15 | |
| Y | EP 1 770 129 A2 (MOLECULAR PROBES INC [US]) 4 April 2007 (2007-04-04) * paragraphs [0069], [0070], [0071], [0076], [0077], [0084] * ----- | 1,3-15 | |
| A | WO 2019/202327 A1 (UNIV WARWICK [GB]) 24 October 2019 (2019-10-24) * the whole document * ----- | 1-15 | |

The present search report has been drawn up for all claims

| Place of search | Date of completion of the search | Examiner |
|---|---|---|
| The Hague | 2 September 2021 | Nurmi, Jussi |

EPO FORM 1503 03.82 (P04C01)

## ANNEX TO THE EUROPEAN SEARCH REPORT
## ON EUROPEAN PATENT APPLICATION NO.

EP 21 16 2266

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2021

| Patent document cited in search report | | Publication date | Patent family member(s) | | Publication date |
|---|---|---|---|---|---|
| US 2006172315 | A1 | 03-08-2006 | US 2006172315 | A1 | 03-08-2006 |
| | | | US 2009226880 | A1 | 10-09-2009 |
| | | | US 2017016879 | A1 | 19-01-2017 |
| | | | US 2017204370 | A1 | 20-07-2017 |
| | | | US 2019390164 | A1 | 26-12-2019 |
| US 2014127717 | A1 | 08-05-2014 | AU 2013334635 | A1 | 05-03-2015 |
| | | | BR 112015003160 | A2 | 04-07-2017 |
| | | | CN 104704366 | A | 10-06-2015 |
| | | | EP 2912464 | A1 | 02-09-2015 |
| | | | ES 2628529 | T3 | 03-08-2017 |
| | | | JP 6480336 | B2 | 06-03-2019 |
| | | | JP 2016506420 | A | 03-03-2016 |
| | | | US 2014127717 | A1 | 08-05-2014 |
| | | | WO 2014066552 | A1 | 01-05-2014 |
| | | | ZA 201500698 | B | 27-09-2017 |
| EP 1770129 | A2 | 04-04-2007 | DE 20122672 | U1 | 05-04-2007 |
| | | | EP 1770129 | A2 | 04-04-2007 |
| | | | EP 1801165 | A2 | 27-06-2007 |
| WO 2019202327 | A1 | 24-10-2019 | AU 2019256786 | A1 | 05-11-2020 |
| | | | EP 3781700 | A1 | 24-02-2021 |
| | | | JP 2021521799 | A | 30-08-2021 |
| | | | US 2021172002 | A1 | 10-06-2021 |
| | | | WO 2019202327 | A1 | 24-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHEN ; LI.** *J. Environ. Monit.*, 2005, vol. 7, 950-959
- **E. CARVALHO et al.** *Aerobiologia,* 2008, vol. 24, 191-201
- **F. MASOTTI et al.** *Trends in Food Science & Technology,* 2019, vol. 90, 147-156
- Handbook of Electroporation. Springer International Publishing, 2017
- **SCHROTTROF et al.** *Journal of Food Engineering,* 2019, vol. 243, 142-152
- **REINEKE et al.** *Frontiers in Microbiology,* 2015, vol. 6, 400
- **JAEGER et al.** *Innovative Food Science & Emerging Technologies,* 2009, vol. 10 (4), 470-480
- **SCHOTTROFF et al.** *Innovative Food Science & Emerging Technologies,* 2020, vol. 63, 102372